# EUROPEAN PATENT SPECIFICATION

(11) **EP 2 400 025 B1**
(45) Date of publication and mention of the grant of the patent: **20.11.2013**
(21) Application number: 10743529.9
(22) Date of filing: 10.02.2010
(51) Int. Cl.: C12P 7/02, C12P 7/22

(54) **Process for producing microbial fermentation product**
Verfahren zur Herstellung eines mikrobiellen Gärungsprodukts
Procédé de production d'un produit de fermentation bactérienne

(30) Priority: 20.02.2009 JP 2009038133
(43) Date of publication of application: 28.12.2011
(73) Proprietor: Kao Corporation, Chuo-Ku Tokyo 103-8210 (JP)
(72) Inventor: HAMADA, Saki, Kamisu-shi Ibaraki 314-0103 (JP); KOYAMA, Shingo, Kamisu-shi Ibaraki 314-0103 (JP); ONOZUKA, Kazuhiro, Kamisu-shi Ibaraki 314-0103 (JP); WATANABE, Takaaki, Kamisu-shi Ibaraki 314-0103 (JP)
(74) Representative: HOFFMANN EITLE
(86) International application number: PCT/JP2010/000843
(87) International publication number: WO 2010/095399

(56) References cited:
- EP-A1- 0 419 026
- WO-A1-01/09074
- WO-A1-02/072110
- WO-A1-2008/108101
- JP-A- 3 224 478
- JP-A- 4 282 319
- JP-A- 2008 212 087
- JP-T- 2003 518 366
- JP-T- 2004 521 137
- JP-T- 2005 503 363

## Description

### FIELD OF THE INVNETION

The present invention relates to a process for producing 1-(2-hydroxyethyl)-2,5,5,8a-tetramethyldecahydronaphthalen-2-ol which is useful as an intermediate for the production of 3a,6,6,9a-tetramethyldodecahydronaphtho[2,1-b]furan.

### BACKGROUND OF THE INVENTION

3a,6,6,9a-Tetramethyldodecahydronaphtho[2,1-b]furan (hereinafter, indicated as "compoundA") is a fragrance component that is contained in ambergris which is a pathologi28cal secretion produced in the body of sperm whale, and is an important compound indispensable as an amber-based perfume. CompoundA is produced mainly by a chemical synthesis method using sclareol, which is extracted from clary sage (*Salvia sclarea* L.), as a starting material. As an intermediate of compound A, 3a,6,6,9a-tetramethyldecahydronaphtho[2,1-b]furan-2(1H)-one (hereinafter, indicated as "sclareolide") and 1-(2-hydroxyethyl)-2,5,5,8a-tetramethyldecahydronaphthalen-2-ol (hereinafter, indicated as "diol") are known.
However, the chemical synthesis method described above has a problem that the environmental burden of the method is heavy, and the yield or the purity cannot be sufficiently obtained.

Meanwhile, there have been reported methods for producing the compound A by obtaining an intermediate of the compound A from sclareol by microbial conversion and cyclizing the intermediate (for example, Patent Documents 1 and 2). Specifically, in the Patent Documents 1 and 2, separation and purification of the diol obtained by microbial conversion is carried out by subjecting a culture fluid to solvent extraction using ethyl acetate, subsequently drying the extract obtained therefrom, dissolving the extract in warm hexane/ethyl acetate or hexane/chloroform, and crystallizing the diol from the solution.
Furthermore, in regard to the separation and purification of the diol obtained from sclareol by microbial conversion, there has been reported a method of filtering a culture fluid using a filter having a mesh size of a specific range to separate the bacterial cells, subsequently dissolving the bacterial cells in a solvent having an SP value of 8.3 to 20, and filtering the solution again (Patent Document 3).

### PRIOR ART DOCUMENTS

### PATENT DOCUMNET

Patent Document 1: JP-A-3-224478
Patent Document 2: JP-A-62-74281
Patent Document 3: JP-A-2008-212087

EP-A-0 419 026 relates to a process and means for producing diol and lactone, such as 1-(2-hydroxyethyl)-2,5,5,8a-tetramethyldecahydronaphthalen-2-ol. The means comprises a microorganism selected from the group consisting of (i) Cryptococcus albidus (Saito [Skinner var. albidus], ATCC 20918; (ii) Bensingtonia ciliata, ATCC 20919; (iii) Cryptococcus laurentii, ATCC 20920; and (iv) Cryptococcus albidus, ATCC 20921, or mixtures of two or more thereof.

EP-A-2 123 765 relates to a method for production of 1-(2-hydroxyethyl)-2,5,5,8a-tetramethyldecahydronaphthalen-2-ol comprising a culturing and a purification step. The culture fluid obtained after cultivation is filtered, the residue washed with water or a solvent having a specific SP value, the obtained cake is dissolved in a solvent having a particular SP value and finally the solution is filtered or centrifuged.

WO-A-02/072110 discloses a method for producing a polyol compound (caloporoside) comprising a culturing and a purification step. After culturing the microbial culture is lyophilized, then extracted with a water-miscible organic solvent such as methanol or propanol, subsequently filtered to remove the residues and separated by column chromatography.

JP-A- 4 282319 discloses a method wherein a culture product from cultured lichen cells is freeze-dried and extracted with a polar solvent at a low temperature.

WO-A-01/09074 relates to a process for the recovery of an organic acid from a fermentation broth which comprises drying the fermentation broth to obtain a dried product, adding the dried product to a lower alcohol in the presence of an acid, and then removing the insoluble material to obtain an organic acid.

### SUMMARY OF INVENTION

### PROBLEM TO BE SOLVED BY THE INVENTION

However, it is known that the crystallization products of the diol obtained by solvent extraction methods using ethyl acetate or the like as described above, have a high intensity of abnormal odor. Particularly, since a culture odor caused by the microorganism is extracted by the solvent, even if the diol is obtained by crystallization, various purification processes are further needed to reduce the culture odor. Thus, it was found that the production process becomes very complicated. Furthermore, the method of separating the bacterial cells with a filter, dissolving the bacterial cells in a particular solvent, and filtering the solution, requires filtration twice, and this method also involves a complicated production step.
On the other hand, even though the intensity of abnormal odor could be decreased, if the recovery rate of the diol itself is too low, the production efficiency of the diol decreases, and the diol cannot be produced efficiently with high purity.

Therefore, the present invention is to provide a method which is capable of efficiently producing the diol with a low intensity of abnormal odor and high purity.

### MEANS FOR SOLVING THE PROBLEM

Conventionally, in case where useful substances that are sparingly water-soluble or hydrophobic are produced outside the bacterial cells, a hydrophobic solvent is added to a culture fluid containing the useful substances, and recovery of the useful substances is carried out through classification or extraction in view of the operation efficiency. However, in practice, when the diol is produced by microbial conversion as described above, the culture fluid contains unreacted sclareol or sclareolide, microorganisms, medium components and the like present in mixture, in addition to the diol. Therefore, the components other than the diol are also recovered at the same time during the classification or extraction, and the diol thus obtained has a high intensity of abnormal odor or a high degree of coloration.
Thus, the inventors of the present invention conducted a thorough investigation on the method for producing the diol. Consequently, the inventors found that when a culture fluid containing the diol is dried before being brought into contact with a solvent to obtain a dried product of the culture fluid, subsequently the dried product is brought into contact with such a solvent that the SP value of the medium obtainable after the contact of the dried product and the solvent is regulated to a specific range, subsequently the bacterial cells are removed, and crystallization is carried out; the diol can be obtained with high yield. The inventors also found that the diol thus obtained has a lowered level of the microbial-derived culture odor or coloration, so that the subsequent processes such as deodorization or repurification can be reduced.

That is, the present invention is to provide a process for producing
1-(2-hydroxyethyl)-2,5,5,8a-tetramethyldecahydronaphthalen-2-ol represented by formula (2):

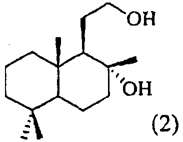

the process comprising culturing a microorganism using a medium containing a compound(s) represented by formula (1a) and/or (1b) 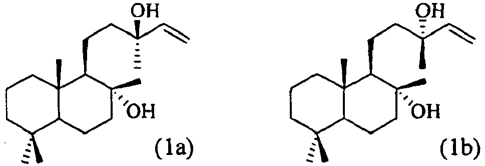 as a substrate, drying the culture fluid containing the diol so that the moisture content in the dried product of the culture fluid is 0.01% to 20%, adding a solvent to the dried product of the culture fluid , whereby the SP value of the medium is adjusted to fall within the range of 9.5 to 16 [(cal/cm³)^{1/2}], thereby obtaining a dispersion, subsequently removing the microorganism from the dispersion, and crystallizing the diol.

### EFFECTS OF THE INVENTION

According to the present invention, the diol, which is useful as an intermediate for the production of the compound A, can be efficiently produced as a high quality product having an excellent odor and color tone.

### MODES FOR CARRYING OUT THE INVENTION

In the present invention, the microorganism that can be utilized in the microbial conversion is not particularly limited as long as it is a microorganism having an ability to produce the diol which is an intermediate of the compound A, by using the compound(s) represented by the formula (1a) and/or formula (1b) as a substrate, and to output the diol outside the bacterial cells. For example, microorganisms belonging to the class Ascomycetes, microorganisms belonging to the genus *Cryptococcus,* microorganisms belonging to the class Basidiomycetes, microorganisms belonging to the genus *Hyphozyma,* and the like may be mentioned. Among these, microorganisms belonging to the class Ascomycetes and microorganisms belonging to the genus *Hyphozyma* are preferred, from the viewpoint of the production efficiency for the diol, which is an intermediate of the compound A. An example of the microorganisms belonging to the class Ascomycetes may include a microorganism designated as Ascomycete sp. KSM-JL2842 and deposited with the International Patent Organism Depositary at the National Institute of Advanced Industrial Science and Technology (address: Central 6, 1-1-1, Higashi, Tsukuba-shi, Ibaraki-ken) on January 12, 2006, under the Accession No. FERM BP-10713. An example of the microorganisms belonging to the genus *Hyphozyma* may include the strain ATCC20624 described in Japanese Patent No. 2547713.

The microorganism that can be utilized in the microbial conversion can be isolated from soil by evaluating the ability of microorganism to produce the diol, which is an intermediate of the compound A, as an indicator. The ability to produce the diol, which is an intermediate of the compoundA, can be evaluated by culturing a test microorganism in a culture medium containing the compound(s) represented by the formula (1a) and/or formula (1b), and detecting the diol, which is an intermediate of the compound A, contained in the medium. Detection of the diol, which is an intermediate of the compound A, can be carried out using conventionally known analysis methods such as gas chromatography (GC), gas-liquid chromatography (GLC), thin layer chromatography (TLC), high performance liquid chromatography (HPLC), infrared spectroscopy (IR), and nuclear magnetic resonance (NMR).

There are no particular limitations imposed on the culture conditions upon the microbial conversion, and a medium of any composition can be used as long as the medium contains the compound(s) represented by the formula (1a) and/or formula (1b) and enables growth of the microorganism. Examples of media that can be used include solid media and liquid media, which respectively contain carbon sources such as monosaccharides, disaccharides, oligosaccharides, polysaccharides, and organic acid salts; nitrogen sources such as inorganic and organic ammonium salts, nitrogen-containing organic substances, and amino acids; minerals such as sodium chloride, ferrous sulfate, magnesium sulfate, manganese sulfate, zinc sulfate, and calcium carbonate; vitamins; and the like. Furthermore, the medium may also contain a surfactant or a defoaming agent in accordance with the culture conditions and the like.

There are no particular limitations on the optimum pH range and the optimum temperature range in terms of the culture conditions. The optimum pH range is preferably pH 3 to 8, more preferably pH 4 to 8, and even more preferably pH 5 to 7, and the optimum temperature as the temperature of the fluid is 10°C to 35°C, more preferably 15°C to 30°C, and even more preferably 20°C to 30°C. The duration of culture is not particularly limited, and is preferably 1 to 10 days from the addition of the substrate. Culture can be carried out using shaking culture, aerobic culture, agitated culture, anaerobic culture, static culture and culture using a fermentation bed, as well as a resting cell reaction and an immobilized cell reaction.

The concentration of the compound(s) represented by the formula (1a) and/or formula (1b) that is added to the medium as a substrate, is preferably set to 0.1% to 50% by mass (hereinafter, simply described as "%") in the medium, from the viewpoint of the production efficiency for the diol, which is an intermediate of the compound A. The substrate may be added to the medium prior to the culture, or may be added in the middle of the culture.

According to the present invention, first, a culture fluid containing the diol represented by the formula (2), which has been extracellularly produced us ing the compound (s) represented by the formula (1a) and/or formula (1b) as a substrate and using the microorganism, is obtained. Furthermore, the culture fluid obtained by microbial conversion is dried, and a dried product of the culture fluid is obtained. After that, a solvent is added to the dried product of the culture fluid thus obtained, and the SP value of the medium obtainable after the contact of the dried product of the culture fluid with the solvent is adjusted to fall in the range of 9.5 to 16 [(cal/cm³)^{1/2}] (hereinafter, the unit will not be described). When the SP value of the medium obtainable after the contact of the dried product of the culture fluid with the solvent is adjusted to preferably 9.5 to 14.5, more preferably 11 to 14, and even more preferably 12 to 13.5, it is advantageous from the viewpoints of improving the solubility of the diol, the recovery ratio (yield) of the diol, and the odor or color of the diol produced. Here, the medium contains the solvent that is used, and the moisture contained in the dried product of the culture fluid. Furthermore, the SP value represents the solubility parameter, and SP values are described in, for example, "Fundamentals and Applications of SP Values and Calculation Methods" (Johokiko Co., Ltd. , 2005), Polymer Handbook Third Edition (A Wiley-Interscience Publication, 1989), and the like. Furthermore, for the solvents whose specific values of the SP value are not described in the foregoing document, the SP values can be determined by using the Fedors method described in, for example, "Fundamentals and Applications of SP Values and Calculation Methods", Polymer Engineering and Science, Vol. 14, No. 2, 147-154 (1974), and the like. In the case of using plural solvents in combination, the SP value is determined by calculating a volume average value of the values of the respective solvents. Furthermore, since the medium obtainable after the contact of the dried product of the culture fluid with the solvent contains the solvent used and moisture originating from the dried product of the culture fluid, the SP value of the medium can be determined similarly by calculating a volume average value from the solvent whose SP values are known and the amount of moisture in the dried product of the culture fluid.

There are no particular limitations on the means for drying the culture fluid obtainable by microbial conversion as long as the drying means is capable of reducing the moisture content in the culture fluid, and examples of the drying means include spray drying, hot air drying, room temperature (10°C to 40°C) drying, heat conduction drying, fluidized drying, rotary drum drying, freeze drying, flash drying, drying under reduced pressure, and the like. Among these, spray drying, hot air or room temperature drying, and rotary drum drying are preferred from the viewpoints of reducing the microbial-derived culture odor and coloration, and spray drying, hot air drying, and rotary drum drying are more preferred and spray drying is even more preferred from the viewpoint of shortening the drying time.
The drying temperature at the time of heating is preferably 40°C to 150°C, from the viewpoints of shortening the drying time and preventing worsening of the odor. In the case of spray drying, the drying temperature is more preferably 50°C to 140°C, and even more preferably 60°C to 130°C, from the viewpoints of shortening the drying time and preventing worsening of the odor. In the case of drying means other than spray drying, the drying temperature is more preferably 40°C to 120°C, even more preferably 50°C to 100°C, and even more preferably 60°C to 80°C.
The moisture content in the dried product of the culture fluid is preferably 0.01% to 20%, and from the viewpoint of the solubility of the diol and from the viewpoints of the recovery rate (yield) of the diol and the reduction of the microbial-derived culture odor and coloration, it is advantageous to adjust the moisture content to more preferably 0.05% to 10%, even more preferably 0.1% to 5%, and even more preferably 0.15% to 3%. The moisture content can be adjusted by drying the culture fluid, but it is also acceptable to further adjust the moisture content of the dried product by adding an appropriate amount of water after drying.

Subsequently, the dried product of the culture fluid is brought into contact with the solvent through mixing or the like to be suspended in the solvent, and the diol is dissolved in the solvent. Concerning the culture fluid or the dried product of the culture fluid, from the viewpoint of suppressing the incorporation of impurities that are present inside the bacterial cells into the solvent, and thereby ameliorating the odor and color tone of the diol thus produced, it is preferable that the microorganism is not subjected to a physical treatment such as crushing or grinding, a chemical treatment such as a surfactant treatment, a biochemical treatment such as a lytic enzyme, or the like, before the culture fluid or the dried product of the culture fluid is brought into contact with the solvent.

Examples of the solvent that is brought into contact with the dried product of the culture fluid include lower alcohols such as methanol (SP value 14.5), ethanol (SP value 13.0), 1-propanol (SP value 12.0), 2-propanol (SP value 11.5), and 1-pentanol (SP value 10.6); ethyl acetate (SP value 9.1); acetic acid (SP value 10.5); diethylene glycol (SP value 14.6); acetone (SP value 9.9);and the like.
These solvents are preferably used singly or in combination of two or more kinds, with the amounts being appropriately set up, so that the SP value of the medium obtainable after the contact of the dried product of the culture fluid with the solvent falls in the range described above.
Among these, from the viewpoint of the recovery rate (yield) of the diol, or from the viewpoints of improving the purity and ameliorating the odor or color tone, it is preferable to use a solvent having an SP value of 9.5 to 16, more preferably a solvent having an SP value of 11 to 14, and even more preferably a solvent having an SP value of 12 to 13, and more preferably, it is advantageous to use 2-propanol, ethanol, or respective aqueous solutions of these alcohols, and even more preferably ethanol or an aqueous solution of ethanol. The ethanol concentration in the aqueous solution of ethanol is preferably 70% or higher from the viewpoint of the solubility of the diol, and the ethanol concentration is more preferably 85% to 99.5%, even more preferably 90% to 99.5%, even more preferably 95% to 99.5%, and even more preferably 99% to 99.5%.

The medium within the system after the contact of the dried product of the culture fluid with the solvent is preferably such that phase separation of water does not occur, from the viewpoint that a process for separating a solvent phase and an aqueous phase is not necessitated. In the case of using a non-water-soluble solvent which has no compatibility with water, it is preferable to prevent phase separation of the solvent and water by using another solvent in combination.

According to the present invention, it is preferable to set the amount of use of the solvent appropriately in accordance with the SP value of the solvent used, so that the SP value of the medium obtainable after the contact of the dried product of the culture fluid with the solvent falls in the range described above. However, from the viewpoints of the solubility of the diol, the amelioration of the odor and color tone of the diol produced, and the recovery rate (yield) of the diol, the amount of use of the solvent based on 100 g of the dried product of the culture fluid is preferably 60 to 6000 mL, and more preferably 180 to 600 mL. Furthermore, the amount of use of the solvent based on 1 g of the diol present in the dried product of the culture fluid is preferably 1 to 100 mL, more preferably 2 to 50 mL, even more preferably 2 to 25 mL, and even more preferably 3 to 10 mL.
The temperature of the medium obtainable after the contact of the dried product of the culture fluid with the solvent is preferably 0°C to 80°C, and more preferably 20°C to 60°C. At this time, the contact time is preferably 1 to 60 minutes, and more preferably 1 to 45 minutes, and from the viewpoint of the solubility of the diol and from the viewpoint of the ameliorating the odor and color tone of the diol produced, the contact time is even more preferably 5 to 30 minutes.

Subsequently, the microorganism is removed from the dispersion resulting from the contact of the dried product of the culture fluid with the solvent. Examples of the means for removing the microorganism include filtration, centrifugation, or the like.
Centrifugation is carried out using a general centrifuge such as a separation plate type centrifuge, a cylinder type centrifuge or a decanter type centrifuge, and it is preferable to perform fractionation of the supernatant after the treatment. For the conditions of centrifugation, the temperature is preferably 5°C to 60°C, and more preferably 20°C to 30°C, and for example, in the case of a cylinder type centrifuge, the speed of rotation is preferably 2000 to 12000 r/min, more preferably 3000 to 12000 r/min, and even more preferably 5000 to 12000 r/min. At this time, the time is preferably 1 to 30 minutes, more preferably 2 to 10 minutes, and even more preferably 5 to 10 minutes.
Filtration is carried out using a general method such as suction filtration, pressure filtration, centrifugal filtrationornatural filtration, and the filtrate obtainedafter the treatment may be fractionated. Among these methods, suction filtration is preferred. The mesh size of the filter used in the filtration is preferably 0.1 to 10 µm, and more preferably 0.2 to 1 µm, from the viewpoints of the recovery rate of the diol and an improvement of purity. The material of the filter is not particularly limited, and specific examples include filters made of resins such as polypropylene, polyester, and nylon; filters made of ceramics, filters made of metals, filters made of cellulose, and the like.

According to the present invention, a diol having high purity and satisfactory odor and color tone can be obtained with high yield, by performing crystallization from the filtrate or supernatant obtained as described above.

There are no particular limitations on the method of crystallization.
For example, a method of subjecting the filtrate or supernatant obtained as described above to an impurity removal operation such as activated carbon filtration or precision filtration as necessary, and then precipitating crystals of the diol through cooling, concentration, addition of a poor solvent, or the like is preferred. There is also available a method of drying the filtrate or supernatant obtained as described above, subsequently dissolving the dried product in an organic solvent, andprecipitatingcrystals of the diol by cooling, concentration, addition of a poor solvent, or the like. The crystals that are obtained in this manner may be purified by recrystallization.
Specifically, crystals are preferably obtained such that the temperature of the filtrate or supernatant obtained as described above or of the organic solvent in which the precipitated crystals of the diol are dissolved is raised to preferably 20°C to 80°C, and more preferably 40°C to 70°C, the resulting liquid is concentrated (the solvent is distilled off) as necessary, and then preferably crystals are precipitated by allowing the resultant to cool to room temperature or adding a poor solvent such as water. In addition, it is not necessary to perform the temperature elevation and cooling at a constant speed, and once crystals begin to precipitate out, it is preferable to maintain the temperature for a while.
Also, in order to separate the crystals from the organic solvent, it is preferable to carry out filtration, centrifugation or the like by the same means as the methods that are used to remove the microorganism, obtain a filter cake, and appropriately wash the cake with an organic solvent. By drying the cake thus obtained, a diol having reduced abnormal odor can be obtained.

Examples of the organic solvent used in crystallization include alcohols having 1 to 4 carbon atoms, such as methanol, ethanol and isopropanol; and organic solvents such as acetone, tetrahydrofuran, ethyl acetate and acetonitrile. Among these, alcohols having 1 to 4 carbon atoms are preferred, and methanol, ethanol and isopropanol are more preferred, while ethanol is even more preferred.
These organic solvents may be used singly, or two or more kinds may be used in mixture.
In the case of adding a poor solvent, it is preferable to use water.
The amount of use of the water is preferably 0.5 to 10 parts by volume, and more preferably 1 to 5 parts by volume, relative to 1 part by volume of the organic solvent that is used to dissolve the diol. The concentration of the diol in the organic solvent at this time is preferably 10 to 1000 g/L, and more preferably 20 to 500 g/L, and the temperature of the liquid at the time of precipitation is preferably 0°C to 60°C, and more preferably 0°C to 30°C.
In regard to the organic solvent for washing the filter cake, it is preferable to use a solvent having an SP value of 9 or less, from the viewpoints of the ameliorating the odor and color tone of the diol produced and the recovery rate. The amount of the solvent is preferably adjusted to 50 to 300 mL relative to 100 g of the cake, from the viewpoint of improving the purity of the diol. Furthermore, the temperature of the solvent used in washing is preferably set to 10°C or lower.

When the crystallization product is dried, the same drying means as the methods that are used to obtain the dried product of the culture fluid may be used, and the drying temperature at the time of heating is preferably room temperature to 90°C.

According to the present invention, it is advantageous from the viewpoint of production efficiency that the recovery rate (yield) of the diol obtained after the crystallization from the filtrate or supernatant obtained as described is preferably 60% or greater, more preferably 65% or greater, and even more preferably 70% or greater.

The process for producing the diol of the present invention can enhance the production efficiency of the diol since the purification steps for the reduction of abnormal odor or for the reduction of coloration can be simplified due to the diol obtained by the process of the present invention being high quality with low intensity of abnormal odor or a low degree of coloration, and at the same time, since the recovery rate of such a high quality diol is also high. Furthermore, the process for producing diol of the present invention is preferable from the production efficacy since a pretreatment prior to extraction may be omitted as the culture fluid obtainable in the production process is dried, and since the extraction time can be shortened while the amount of the solvent used for extraction canbe reduced.
The diol thus obtained is converted to the compound A through deodorization and cyclization in various solvents, using an acidic catalyst, for example, p-toluenesulfonic acid, p-toluenesulfonic acid chloride, a catalytic amount of sulfuric acid, or an acidic ion exchanger.

### EXAMPLES

Hereinafter, the present invention will be described in detail by way of Examples, but the present invention is not intended to be limited by these Examples.

### [Microbial conversion 1]

One platinum loop of *Hyphozyma roseoniger* strain ATCC20624 (hereinafter, also referred to as "ATCC") was inoculated into 2.1% YM broth, and subjected to shaking culture at 25°C for three days. The resultant was used as an inoculum. Subsequently, the 0.3% of inoculum was inoculated into a medium containing 2.1% YM broth and 0.1% magnesium sulfate, and aerated agitated culture was carried out in a 30-L fermenter at a fluid temperature of 24°C, an amount of aeration of 0.5 vvm, and a stirring speed of 200 r/min for three days. Thereafter, a substrate including 10% Tween 80 (TradeMark) and 20% sclareol was added to the culture to obtain a final sclareol concentration in the culture fluid of 2%. For 4 days from the addition of the substrate, aerated agitated culture was performed while the pH was regulated to 6.0 using 1 N NaOH and 1 N HCl. The resulting culture fluid was designated as culture fluid1. This culture fluid1 contained 1.9% of the diol, 0.1% of sclareol, 97.4% of water, and 0.6% of other solids (bacterial cells and the like).

### [Microbial conversion 2]

The same operation as that performed in the Microbial conversion 1 was carried out, except that the microorganism used was Ascomycete sp. strain KSM-JL2842 (FERM BP-10713) (hereinafter, also referred to as "KSM"), and thus a culture fluid 2 was obtained. The culture fluid 2 contained 1.5% of the diol, 0.3% of sclareol, 97.2% of water, and 1.0% of other solids (bacterial cells and the like).

### [Analysis methods]

Sclareol, sclareolide and the diol were extracted from the culture fluid or a dried product thereof using ethyl acetate, and were appropriately diluted. An analysis by gas chromatography (GC) was performed to measure the contents. The GC analysis was performed with a 6890N GC System (Agilent Technologies, Inc.), and the analysis conditions were as follows. An FID (Flame Ionization Detector) (Agilent Technologies, Inc.) was used as a detector, the injection inlet temperature was set at 250°C, and the injection method was set in the split mode (split ratio 100:1). The total flow was 200 ml/min, the column flow rate was 0.4 ml/min, the column was DB-WAX (φ0.1 mm x 10 m) (J&W Technology, Ltd.), and the oven temperature was 250°C.
The amount of water in the culture fluid was calculated from the amount of mass reduction after the culture fluid was dried for 2 hours using an electric dryer at 120°C. Furthermore, the moisture content in the dried product was measured using a Hiranuma moisture content meter, AQ-7 (Hiranuma Sangyo Corp.).

### [Method for evaluation of odor]

The odor evaluation of the crystals of the diol was performed by eight panels according to the criteria shown below, and the average value was designated as the odor evaluation value.
5 : A strong odor of the microbial culture fluid remains.
4: A slightly strong odor of the microbial culture fluid remains.
3: The odor of the microbial culture fluid is weak.
2 : The odor of the microbial culture fluid is negligible.
1: There is no odor of the microbial culture fluid.

### [Method for evaluation of color of extract liquid]

The evaluation of the color of the diol extract liquid was carried out such that the diol was dissolved in a solvent, the microorganism was removed by centrifugation, the absorbance of the supernatant thus obtained was measured at a wavelength of 420 nm, and the color value was calculated by dividing the absorbance by the concentration (g/L) of the diol. A smaller value resulted in more satisfactory color tone.

### [Method for evaluation of color of crystals]

The color of crystals of the diol was measured using a color measurement colorimeter ZE-2000 type (Nippon Denshoku Industries Co., Ltd.). As the value representing a yellowish tinge (value b) was smaller, the color tone was more satisfactory.

### Example 1

The culture fluid 1 was spray dried at 130°C, and thus a dried product having a moisture content of 2% was obtained. 5 g of the dried product of culture fluid 1 was added to 15 mL of ethanol (99.5%, SP value 13.0), and the mixture was mixed for 10 minutes (temperature of the liquid 20°C) to dissolve the diol. Microorganisms were removed from the liquid by centrifugation (5000 r/min, 5 minutes), and the liquid was filtered through a 0.2-µm PTFE filter. Subsequently, 30 mL of water was added to 11 mL of the filtrate thus obtained to precipitate the diol, and the liquid was filtered through a No.2 filter paper. The temperature of the liquid at the time of precipitation was 25°C. Subsequently, the precipitate was dried at 80°C, and thus crystals of the diol were obtained.
In order to precipitate the diol from the filtrate, the concentration of the diol in the dried product of culture fluid and the solubility of the diol in a solvent were measured in advance according to the analysis methods described above, and the amount of water to be added so as to precipitate almost the entire amount of the diol was determined (the same applies in the following Examples).

### Examples 2 and 3

The culture fluid 1 was spray dried at 130°C, and two dried products having a moisture content of 2% were obtained. Water was added to the respective driedproducts to adjust the moisture content to 5% and 10%, and thus dried products having moisture contents of 5% and 10% were produced.
5 g of the dried product having the moisture content adjusted to 5% was added to 15 mL of ethanol, and the mixture was mixed for 10 minutes (temperature of the liquid 20°C) to dissolve the diol. Microorganisms were removed from the liquid by centrifugation (5000 r/min, 5 minutes), and the liquid was filtered through a 0.2-µm PTFE filter. Subsequently, 30 mL of water was added to 11 mL of the filtrate thus obtained to precipitate the diol, and the liquid was filtered through a No.2 filter paper. The temperature of the liquid at the time of precipitation was 25°C. Subsequently, the precipitate was dried at 80°C, and thus crystals of the diol were obtained (Example 2).
Crystals of the diol were obtained from the dried product having the moisture content adjusted to 10% (5 g), in the same manner as described above (Example 3).

### Example 4

The culture fluid 1 was hot air dried at 80°C with an electric dryer, and a dried product having a moisture content of 5% was obtained. 5 g of the dried product of culture fluid was added to 15 mL of ethanol, and the mixture was mixed for 10 minutes (temperature of the liquid 20°C) to dissolve the diol. Microorganisms were removed from the liquid by centrifugation (5000 r/min, 5 minutes), and the liquid was filtered through a 0.2-µm PTFE filter. Subsequently, 30 mL of water was added to 11 mL of the filtrate thus obtained to precipitate the diol, and the liquid was filtered through a No.2 filter paper. The temperature of the liquid at the time of precipitation was 25°C. Subsequently, the precipitate was dried at 80°C, and thus crystals of the diol were obtained.

### Example 5

The culture fluid 1 was dried at room temperature of 25°C, and a dried product having a moisture content of 5% was obtained. 5 g of the dried product of culture fluid was added to 15 mL of ethanol, and the mixture was mixed for 10 minutes (temperature of the liquid 20°C) to dissolve the diol. Microorganisms were removed from the liquid by centrifugation (5000 r/min, 5 minutes), and the liquid was filtered through a 0.2-µm PTFE filter. Subsequently, 30 mL of water was added to 11 mL of the filtrate thus obtained toprecipitate the diol, and the liquidwas filtered through a No. 2 filter paper. The temperature of the liquid at the time of precipitation was 25°C. Subsequently, the precipitate was dried at 80°C, and thus crystals of the diol were obtained.

### Examples 6 and 7

Crystals of the diol were obtained in the same manner as in Example 1, except that the ethanol to which 5 g of the dried product was added, was changed to 2-propanol (IPA) (Example 6). Furthermore, crystals of the diol were obtained in the same manner as in Example 1, except that ethanol was changed to methanol (Example 7).

### Comparative Example 1

The culture fluid 1 was centrifuged (5000 r/min, 5 min), and the supernatant was removed to obtain the sediment. The sediment was not dried, and the moisture content was 58%. 11.9 g of the sediment of the culture fluid (equivalent to 5 g of a dried product) was added to 15 mL of ethanol, and the mixture was mixed for 10 minutes (temperature of the liquid 20°C) to dissolve the diol. Microorganisms were removed from the liquid by centrifugation (5000 r/min, 5 minutes), and the liquid was filtered through a 0.2-µm PTFE filter. Subsequently, 30 mL of water was added to 11 mL of the filtrate thus obtained to precipitate the diol, and the liquid was filtered through a No.2 filter paper. Subsequently, the precipitate was dried at 80°C, and thus crystals of the diol were obtained.

### Comparative Example 2

Crystals of the diol were obtained in the same manner as in Example 1, except that the ethanol to which 5 g of the dried product was added, was changed to a 65% aqueous solution of ethanol.

### Comparative Example 3

The culture fluid 1 was spray dried at 130°C, and a dried product having a moisture content of 2% was obtained. 5 g of the dried product of culture fluid was added to 15 mL of toluene (SP value 8.9), and the mixture was mixed for 10 minutes (temperature of the liquid 20°C) to dissolve the diol. Microorganisms were removed from the liquid by centrifugation (5000 r/min, 5 minutes), and the liquid was filtered through a 0.2-µm PTFE filter. Subsequently, the filtrate thus obtained was treated such that the solvent was distilled off under reduced pressure at 40°C. The entire amount of the diol was precipitated by evaporation and drying, and thus crystals of the diol were obtained.

The results of Examples 1 to 7 and Comparative examples 1 to 3 are presented in Table 1.

**[Table 1]**

| | | Example 1 | Example 2 | Example 3 | Example 4 | Example 5 | Example 6 | Example 7 | Comparative Example 1 | Comparative Example 2 | Comparative Example 3 |
|---|---|---|---|---|---|---|---|---|---|---|---|
| Raw material | Type of bacterium | ATCC | ATCC | ATCC | ATCC | ATCC | ATCC | ATCC | ATCC | ATCC | ATCC |
| Method | Method for treatment of culture fluid | Spraying | Spraying | Spraying | Hot air drying | Room temperature drying | Spraying | Spraying | Centrifugation | Spraying | Spraying |
| | Moisture content after treatment of culture fluid | 2% | 5% | 10% | 5% | 5% | 2% | 2% | 58% | 2% | 2% |
| | Solvent used | 99.5% EtOH | 99.5% EtOH | 99.5% EtOH | 99.5% EtOH | 99.5% EtOH | IPA | Methanol | 99.5% EtOH | 65% EtOH | Toluene |
| | SP value of medium after contact with culture fluid | 13.1 | 13.2 | 13.3 | 13.2 | 13.2 | 11.6 | 14.6 | 14.7 | 16.7 | 8.9 |
| | Method of crystallization | Addition of water | Addition of water | Addition of water | Addition of water | Addition of water | Addition of water | Addition of water | Addition of water | Addition of water | Drying |
| Evaluation | Recovery rate of diol | 74.6% | 72.1% | 69.1% | 60.3% | 79.8% | 77.0% | 79.8% | 66.2% | 70.7% | 12.5% |
| | Color of extract liquid | 0.0008 | 0.0011 | 0.0018 | 0.0022 | 0.0013 | 0.0002 | 0.0037 | 0.0168 | 0.0059 | 0.0007 |
| | Color of crystals | 3.71 | 5.75 | 6.09 | 4.90 | 5.44 | 7.31 | 13.61 | 14.04 | 15.06 | 12.05 |
| | Odor evaluation | 1 | 2 | 2 | 2 | 2 | 2 | 3 | 3 | 3 | 3 |

### Example 8

The culture fluid 2 was spray dried at 130°C, and a dried product having a moisture content of 2% was obtained. 5 g of the dried product of culture fluid was added to 15 mL of ethanol (SP value 13.0), and the mixture was mixed for 10 minutes (temperature of the liquid 20°C) to dissolve the diol. Microorganisms were removed from the liquid by centrifugation (5000 r/min, 5 minutes), and the liquid was filtered through a 0.2-µm PTFE filter. Subsequently, 30 mL of water was added to 11 mL of the filtrate thus obtained to precipitate the diol, and the liquid was filtered through a No.2 filter paper. The temperature of the liquid at the time of precipitation was 25°C. Subsequently, the precipitate was dried at 80°C, and thus crystals of the diol were obtained.

### Examples 9 and 10

The culture fluid 2 was spray dried at 130°C, and two dried products having a moisture content of 2% were obtained. Water was added to the respective dried products to adjust the moisture content to 5% and 10%, and thus dried products having moisture contents of 5% and 10% were produced.
5 g of the dried product having the moisture content adjusted to 5% was added to 15 mL of ethanol, and the mixture was mixed for 10 minutes (temperature of the liquid 20°C) to dissolve the diol. Microorganisms were removed from the liquid by centrifugation (5000 r/min, 5 minutes), and the liquid was filtered through a 0.2-µm PTFE filter. Subsequently, 30 mL of water was added to 11 mL of the filtrate thus obtained to precipitate the diol, and the liquid was filtered through a No.2 filter paper. The temperature of the liquid at the time of precipitationwas 25°C . Subsequently, the precipitate was dried at 80°C, and thus crystals of the diol were obtained (Example 9).
Crystals of the diol were obtained from the dried product having the moisture content adjusted to 10% (5 g), in the same manner as described above (Example 10).

### Example 11

The culture fluid 2 was hot air dried at 8 0°C with an electric dryer, and a dried product having a moisture content of 4% was obtained. 5 g of the dried product of culture fluid was added to 15 mL of ethanol, and the mixture was mixed for 10 minutes (temperature of the liquid 20°C) to dissolve the diol. Microorganisms were removed from the liquid by centrifugation (5000 r/min, 5 minutes), and the liquid was filtered through a 0.2-µm PTFE filter. Subsequently, 30 mL of water was added to 11 mL of the filtrate thus obtained to precipitate the diol, and the liquid was filtered through a No.2 filter paper. The temperature of the liquid at the time of precipitation was 25°C. Subsequently, the precipitate was dried at 80°C, and thus crystals of the diol were obtained.

### Example 12

The culture fluid 2 was dried at room temperature of 25°C, and a dried product having a moisture content of 4% was obtained. 5 g of the dried product of culture fluid was added to 15 mL of ethanol, and the mixture was mixed for 10 minutes (temperature of the liquid 20°C) to dissolve the diol. Microorganisms were removed from the liquid by centrifugation (5000 r/min, 5 minutes), and the liquid was filtered through a 0.2-µm PTFE filter. Subsequently, 30 mL of water was added to 11 mL of the filtrate thus obtained to precipitate the diol, and the liquid was filtered through a No.2 filter paper. The temperature of the liquid at the time of precipitation was 25°C. Subsequently, the precipitate was dried at 80°C, and thus crystals of the diol were obtained.

### Comparative Examples 4 to 6

The culture fluid 2 was spray dried at 130°C, and a dried product having a moisture content of 2% was obtained. 5-g portions of the dried product of culture fluid were respectively added to 15 mL each of various solvents such as toluene (SP value 8.9), 65% ethanol (SP value 16.7) and hexane (SP value 7.3), and the mixtures were mixed for 10 minutes (temperature of the liquid 20°C) to dissolve the diol. Microorganisms were removed from the liquids by centrifugation (5000 r/min, 5 minutes), and each of the liquids was filtered through a 0.2-µm PTFE filter. Subsequently, from each of the filtrates thus obtained, the solvent was distilled off under reduced pressure at 40°C to precipitate the entire amount of the diol, and thus crystals of the diol were obtained. In Comparative Example 6, since the yield of the diol was very low, an evaluation of the color of the crystals was not carried out.

### Comparative Example 7

The culture fluid 2 was centrifuged (5000 r/min, 5 min), and the supernatant was removed to obtain a sediment. The sediment was not dried, and the moisture content was 57%. 11.6 g of the sediment of the culture fluid (equivalent to 5 g of a dried product) was added to 15 mL of ethanol, and the mixture was mixed for 10 minutes (temperature of the liquid 20°C) to dissolve the diol. Microorganisms were removed from the liquid by centrifugation (5000 r/min, 5 minutes), and the liquid was filtered through a 0.2-µm PTFE filter. Subsequently, 30 mL of water was added to 11 mL of the filtrate thus obtained to precipitate the diol, and the liquid was filtered through a No.2 filter paper. Subsequently, the precipitate was dried at 80°C, and thus crystals of the diol were obtained.

The results of Examples 8 to 12 and Comparative examples 4 to 7 are presented in Table 2.

**[Table 2]**

| | | Example 8 | Example 9 | Example 10 | Example 11 | Example 12 | Comparative Example 4 | Comparative Example 5 | Comparative Example 6 | Comparative Example 7 |
|---|---|---|---|---|---|---|---|---|---|---|
| Raw material | Type of bacterium | KSM | KSM | KSM | KSM | KSM | KSM | KSM | KSM | KSM |
| Method | Method for treatment of culture fluid | Spray drying | Spray drying | Spray drying | Hot air drying | Room temperature drying | Spray drying | Spray drying | Spray drying | Centrifugation |
| | Moisture content after treatment of culture fluid | 2% | 5% | 10% | 4% | 4% | 2% | 2% | 2% | 37% |
| | Solvent used | 99.5% EtOH | 99.5% EtOH | 99.5% EtOH | 99.5% EtOH | 99.5% ETOH | Toluene | 65% EtOH | Hexane | 99.5% EtOH |
| | SP value of medium after contact with culture fluid | 13.1 | 13.2 | 13.3 | 13.1 | 13.1 | 8.9 | 16.7 | 7.3 | 14.7 |
| | Method of crystallization | Addition of water | Addition of water | Addition of water | Addition of water | Addition of water | Drying to solid | Drying to solid | Drying to solid | Addition of water |
| Evaluation | Recovery rate of diol | 93.5% | 88.6% | 85.9% | 81.8% | 71.1% | 19.3% | 56.7% | 3.1% | 87.3% |
| | Color of extract liquid | 0.0006 | 0.0007 | 0.0011 | 0.0016 | 0.0013 | 0.0017 | 0.0057 | 0.0043 | 0.0044 |
| | Color of crystals | 4.21 | 5.82 | 6.77 | 5.96 | 5.52 | 5.2 | 21.4 | - | 9.89 |
| | Oder evaluation | 1 | 2 | 2 | 2 | 2 | 3 | 5 | 2 | 3 |

From the results of Table 1 and Table 2, when a product obtained by drying a culture fluid was brought into contact with a solvent, the SP value of the medium was adjusted to the range of 9.5 to 16, and a filtrate obtained after removal of the microorganism was subjected to a crystallization process, a high quality diol having satisfactory odor and color tone could be obtained, and the recovery rate of the diol was also high. Furthermore, when the culture fluid was not dried and was directly brought into contact with a solvent, even if the SP value of the medium obtainable after the contact of the culture fluid and the solvent was adjusted to the range of 9.5 to 16, a high quality product could not be obtained.

## Claims

1. A process for producing
1-(2-hydroxyethyl)-2,5,5,8a-tetramethyldecahydronaphthalen-2-ol represented by formula (2): 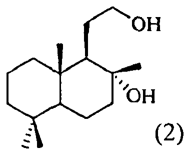 the process comprising culturing a microorganism using a medium containing a compound(s) represented by formula (1a) and/or (1b) 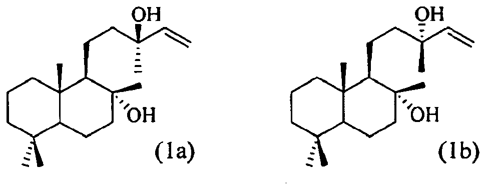 as a substrate, drying the culture fluid containing the diol so that the moisture content in the dried product of the culture fluid is 0.01% to 20%, adding a solvent to the dried product of the culture fluid , whereby the SP value of the medium is adjusted to fall within the range of 9.5 to 16 [(cal/cm³)^{1/2}], thereby obtaining a dispersion, subsequently removing the microorganism from the dispersion, and crystallizing the diol.

2. The process according to claim 1, wherein, after the culture fluid is dried, the solvent is added to the dried product of the culture fluid without treating the microorganism by a physical treatment, a chemical treatment or a biochemical treatment.

3. The process according to claim 1 or claim 2, wherein the amount of solvent added to the dried product of the culture fluid is 1 to 100 mL relative to 1 g of 1-(2-hydroxyethyl)-2,5,5,8a-tetramethyldecahydronaphthalen-2-ol present in the dried product of the culture fluid.

4. The process according to any one of claims 1 to 3, wherein the contact time of the dried product of the culture fluid and the solvent is 1 to 60 minutes.

## Patentansprüche

1. Verfahren zur Herstellung von 1-(2-Hydroxyethyl)-2,5,5,8a-tetramethyldecahydronaphthalin-2-ol der Formel (2): 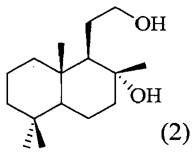 wobei das Verfahren die Kultivierung eines Mikroorganismus unter Verwendung eines Mediums, das (eine) Verbindung(en) der Formel (1a) und/oder (1b) enthält: 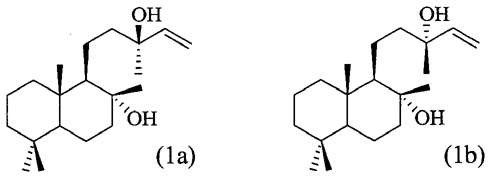 als Substrat, Trocknen der Kulturflüssigkeit, die das Diol enthält, so dass der Feuchtigkeitsgehalt in dem getrockneten Produkt der Kulturflüssigkeit 0,01 bis 20 % beträgt, Zugabe eines Lösungsmittels zu dem getrockneten Produkt der Kulturflüssigkeit, wodurch der SP-Wert des Mediums so eingestellt wird, dass er in den Bereich von 9,5 bis 16 [(cal/cm³)^{1/2}] fällt, wodurch eine Dispersion erhalten wird, anschliessendes Entfernen des Mikroorganismus aus der Dispersion und Kristallisieren des Diols umfasst.

2. Verfahren gemäss Anspruch 1, wobei nach dem Trocknen der Kulturflüssigkeit das Lösungsmittel zu dem getrockneten Produkt der Kulturflüssigkeit ohne Behandlung des Mikroorganismus durch eine physikalische Behandlung, eine chemische Behandlung oder eine biochemische Behandlung zugegeben wird.

3. Verfahren gemäss Anspruch 1 oder Anspruch 2, wobei die Menge des zu dem getrockneten Produkt der Kulturflüssigkeit zugegeben Lösungsmittels 1 bis 100 ml in bezug auf 1 g 1-(2-Hydroxyethyl)-2,5,5,8a-tetramethyldecahydronaphthalin-2-ol, das in dem getrockneten Produkt der Kulturflüssigkeit vorhanden ist, beträgt.

4. Verfahren gemäss irgendeinem der Ansprüche 1 bis 3, wobei die Kontaktzeit des getrockneten Produkts der Kulturflüssigkeit und des Lösungsmittels 1 bis 60 Minuten beträgt.

## Revendications

1. Procédé de production de 1-(2-hydroxyéthyl)-2,5,5,8a-tétraméthyldécahydronaphthalén-2-ol représenté par la formule (2) : 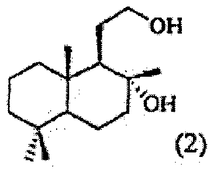 le procédé comprenant la culture d'un micro-organisme en utilisant un milieu contenant un(des) composé(s) représenté(s) par la formule (1a) et/ou (1b) 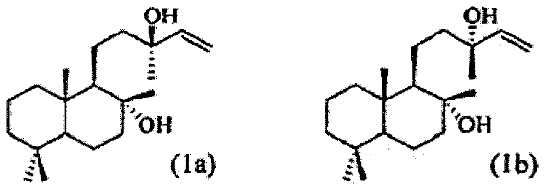 en tant que substrat, le séchage du fluide de culture contenant le diol de sorte que la teneur en humidité dans le produit séché du fluide de culture soit de 0,01 % à 20 %, l'ajout d'un solvant au produit séché du fluide culture, moyennant quoi la valeur SP du milieu est ajustée pour entrer dans la plage de 9,5 à 16 [(cal/cm³)^{1/2}], obtenant ainsi une dispersion, l'élimination par la suite du micro-organisme de la dispersion, et la cristallisation du diol.

2. Procédé selon la revendication 1, dans lequel, après que le fluide de culture soit séché, le solvant est ajouté au produit séché du fluide de culture sans traitement du micro-organisme par un traitement physique, un traitement chimique ou un traitement biochimique.

3. Procédé selon la revendication 1 ou la revendication 2, dans lequel la quantité de solvant ajoutée au produit séché du fluide de culture est de 1 à 100 ml par rapport à 1 g de 1-(2-hydroxyéthyl)-2,5,5,8a-tétraméthyldécahydronaphthalén-2-ol présent dans le produit séché du fluide de culture.

4. Procédé selon l'une quelconque des revendications 1 à 3, dans lequel le temps de contact du produit séché du fluide de culture et du solvant est de 1 à 60 minutes.
